(19) Europäisches Patentamt
European Patent Office
Office européen des brevets

(11) Veröffentlichungsnummer: **0 234 516 B1**

(12) # EUROPÄISCHE PATENTSCHRIFT

(45) Veröffentlichungstag der Patentschrift: **29.04.92**

(51) Int. Cl.$^5$: **C07D 471/04**, A61K 31/435, //(C07D471/04,221:00,221:00)

(21) Anmeldenummer: **87102398.2**

(22) Anmeldetag: **20.02.87**

Die Akte enthält technische Angaben, die nach dem Eingang der Anmeldung eingereicht wurden und die nicht in dieser Patentschrift enthalten sind.

(54) **1,6-Naphthyridin-Derivate,Verfahren zu deren Herstellung und diese enthaltende Arzneimittel zur Behandlung von Gefässerkrankungen.**

(30) Priorität: **22.02.86 DE 3605743**

(43) Veröffentlichungstag der Anmeldung:
**02.09.87 Patentblatt 87/36**

(45) Bekanntmachung des Hinweises auf die Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**EP-A- 0 133 530**
**EP-A- 0 173 933**

(73) Patentinhaber: **GÖDECKE AKTIENGESELLSCHAFT**
**Salzufer 16**
**W-1000 Berlin 10(DE)**

(72) Erfinder: **Kleinschroth, Jürgen, Dr.**
**Ricarda-Huch-Strasse 11**
**W-7809 Denzlingen(DE)**
Erfinder: **Mannhardt, Karl, Dr.**
**Pfauenstrasse 14**
**W-7807 Elzach-Oberprechtal(DE)**
Erfinder: **Hartenstein, Johannes, Dr.**
**Fohrenbühl 23**
**W-7801 Stegen-Wittental(DE)**
Erfinder: **Satzinger, Gerhard, Dr.**
**Im Mattenbühl 7**
**W-7809 Denzlingen(DE)**
Erfinder: **Muster, Dieter, Dr.**
**Schrahöfe 9**
**W-7807 Elzach-Oberprechtal(DE)**
Erfinder: **Steinbrecher, Wolfgang, Dr.**
**Schwarzwaldstrasse 16**
**W-7803 Gundelfingen(DE)**
Erfinder: **Wagner, Bernd, Dr.**
**Am Lossele 5**
**W-7809 Denzlingen(DE)**
Erfinder: **Osswald, Hartmut, Dr.**
**Kiefernweg 1**
**W-7808 Waldkirch 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 234 516 B1

## Beschreibung

Aus EP-A-0 133530 und EP-A-0 173 933 sind 1,6-Napthyridin-Derivate zur Behandlung von Gefässerkrankungen bekannt.

Die Erfindung betrifft neue
5-Oxy-substituierte -1,6-Naphthyridin-Derivate, nämlich
(±)-2-Amino-1,4-dihydro-5-isopropoxy-4-(2-trifluormethylphenyl)-1,6-naphthyridin-3-carbonsäureethylester,
(±)-1,4-Dihydro-5-isopropoxy-2-methyl-4-(2-trifluormethylphenyl)-1,6-naphthyridin-3-carbonsäure-(2-[1-(4-diphenylmethylpiperazinyl)]ethyl)ester • 5 Oxalat,
(±)-2-Dimethylaminomethyl-1,4-dihydro-5-isopropoxy-4-(2-trifluormethylphenyl)-1,6-naphthyridin-3-carbonsäureethylester • Dihydrochlorid,
(±)-[3-Ethoxycarbonyl-1,4-dihydro-5-isopropoxy-4-(2-trifluormethylphenyl)-1,6-napthyridin-2-yl]methyl-1-pyridiniumbromid,
(±)-2-(2-Aminoethoxymethyl)-1,4-dihydro-5-isopropoxy-4-(2-trifluormethylphenyl)-1,6-naphthyridin-3-carbonsäureethylester • Hydrochlorid,
(±)-1,4-Dihydro-5-isopropoxy-2-methoxymethyl-4-(2-trifluormethylphenyl)-1,6-naphthyridin-3-carbonsäureethylester,
(±)-1,4-Dihydro-2-hydroxymethyl-5-isopropoxy-4-(2-trifluormethylphenyl)-1,6-naphthyridin-3-carbonsäureethylester,
(±)-1,4-Dihydro-5-isopropoxy-2-phenylthiomethyl-4-(2-trifluormethylphenyl)-1,6-naphthyridin-3-carbonsäureethylester,
(±)-1,4-Dihydro-5-isopropoxy-2-(4-pyridylthiomethyl)-4-(2-trifluormethylphenyl)-1,6-naphthyridin-3-carbonsäureethylester,
(±)-2-(2-N-Benzyl-N-methylaminoethoxymethyl)-1,4-dihydro-5-isopropoxy-4-(2-triflourmethylphenyl)-1,6-napthyridin-3-carbonsäureethylester • 2 Oxalat,
(±)-1,4-Dihydro-5-isopropoxy-2-methyl-4-(2-trifluormethylphenyl)-1,6-naphthyridin-3-carbonsäure-[2-(N-benzyl-N-methylamino)ethyl]-amid • Dihydrochlorid,
(±)-1,4-Dihydro-5-isopropoxy-2-methyl-4-(2-trifluormethylphenyl)-1,6-naphthyridin-3-carbonsäure-N-ethylamid,
(±)-1,4-Dihydro-5-isopropoxy-2-methyl-4-(2-trifluormethylphenyl)-1,6-naphthyridin-3-carbonsäureamid,
(±)-1,4-Dihydro-5-isopropoxy-2-phenylsulfinylmethyl-4-(2-trifluormethylphenyl)-1,6-napthyridin-3-carbonsäureethylester,
(±)-1,4-Dihydro-5-isopropoxy-2-phenylsulfonylmethyl-4-(2-trifluormethylphenyl)-1,6-naphthyridin-3-carbonsäureethylester,
(±)-1,4-Dihydro-5-isopropoxy-2-methyl-4-(2-trifluormethylphenyl)-1,6-napthyridin-3-carbonsäure-(2-aminoethyl)ester,
(±)-2-Formyl-1,4-dihydro-5-isopropoxy-4-(2-trifluormethylphenyl)-1,6-naphthyridin-3-carbonsäureethylester,
(±)-2-Cyano-1,4-dihydro-5-isopropoxy-4-(2-trifluormethylphenyl)-1,6-naphthyridin-3-carbonsäureethylester,
(±)-1,4-Dihydro-5-isopropoxy-2-methyl-4-(2-trifluormethylphenyl)-1,6-naphthyridin-3-carbonitril,
(±)-1,4-Dihydro-5-(1-dimethylamino-2-propoxy)-2-methyl-4-(2-trifluormethylphenyl)-1,6-naphthyridin-3-carbonsäureethylester • Dihydrochlorid,
(±)-1,4-Dihydro-2-(2-hydroxyethoxymethyl)-5-isopropoxy-4-(2-trifluormethylphenyl)-1,6-naphthyridin-3-carbonsäureethylester,
(±)-2-(2-Dimethylaminoethoxymethyl)-1,4-dihydro-5-isopropoxy-4-(1-naphthyl)-1,6-naphthyridin-3-carbonsäureethylester,
(±)-1,4-Dihydro-5-isopropoxy-2-methyl-4-(1-naphthyl)-1,6-naphthyridin-3-carbonsäureethylester,
(±)-[3-Ethoxycarbonyl-1,4-dihydro-5-isopropoxy-4-(1-naphthyl)-1,6-napthyridin-2-yl]methyl-(2-hydroxyethyl)-dimethylammoniumbromid,
(±)-2-Dibrommethyl-1,4-dihydro-5-isopropoxy-4-phenyl-1, 6-naphthyridin-3-carbonsäureethylester,
(±)-1,4-Dihydro-5-isopropoxy-2-[(4-methylpiperazin-1-yl)methyl]-4-phenyl-1,6-naphthyridin-3-carbonsäuremethylester,
(±)-4-(2-Chlorphenyl)-1,4-dihydro-2-hydroxymethyl-5-isopropoxy-1,6-naphthyridin-3-carbonsäureethylester,
(±)-2-(2-Aminoethoxymethyl)-4-(2-chlorphenyl)-1,4-dihydro-5-isopropoxy-1,6-naphthyridin-3-carbonsäureethylester • Dihydrochlorid und
(±)-4-(2-Bromphenyl)-2-dibrommethyl-1,4-dihydro-5-isopropoxy-1,6-naphthyridin-3-carbonsäuremethylester.

Die Erfindung betrifft weiterhin
Verfahren zur Herstellung von 5-oxy-substituierten 1,6-Naphthyridin-Derivaten, dadurch gekennzeichnet, daß man entweder

a) 1,6-Naphthyridinon-Derivate der allgemeinen Formel V

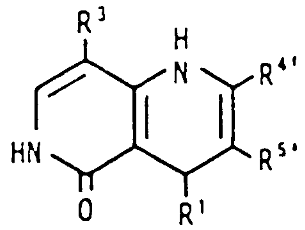

(V)

in welcher $R^1$ und $R^3$ die gemäß der genannten Verbindungen entsprechende Bedeutung haben, $R^{4'}$ eine Methyl-oder Aminogruppe und $R^{5'}$, einen Methoxycarbonyl-, Ethoxycarbonyl- oder N-Diphenylmethylpiperazinylethoxycarbonylrest oder einen Carboxamidrest der allgemeinen Formel IV

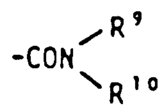

(IV)

worin $R^9$ und $R^{10}$ gleich oder verschieden sein können und Wasserstoff, eine Ethylgruppe oder eine N-Benzyl-N-methylaminoethylgruppe darstellen, bedeutet, in an sich bekannter Weise am Lactamsauerstoffatom alkyliert,
oder
b) 1,6-Naphthyridin-Derivate der allgemeinen Formel Ia

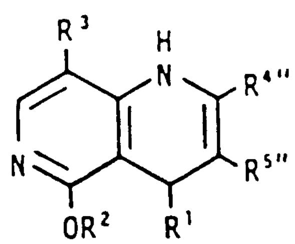

(Ia)

in welcher $R^1$ und $R^3$ die oben angegebene Bedeutung haben, $R^2$ für eine geradkettige oder verzweigte Alkylgruppe mit bis zu 4 Kohlenstoffatomen steht $R^{4''}$ für eine Methylgruppe und R5" für einen Benzyloxycarbonylrest steht, in an sich bekannter Weise hydrogenolytisch zu entsprechenden Carbonsäuren spaltet und diese wiederum in an sich bekannter Weise über die Zwischenstufe eines Säurehalogenides mit entsprechend substituierten Alkoholen oder Aminen zu Ester- bzw. Amidderivaten der genannten 5-oxy-substituierten-1,6-Naphthyridinderivate umsetzt,
oder
c) 1,6-Naphthyridin-Derivate der allgemeinen Formel Ib,

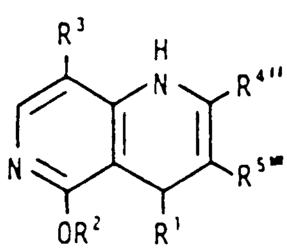

(Ib)

in welcher $R^1$, $R^2$, $R^3$ und $R^{4''}$ die oben angegebene Bedeutung haben und $R^{5'''}$, einen Methoxycarbonyl-, Ethoxycarbonyl- oder N-Diphenylmethylpiperazinylethoxycarbonylrest oder einen Carboxamidrest der

allgemeinen Formel IV

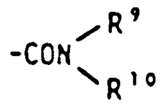

(IV)

worin $R^9$ und $R^{10}$ gleich oder verschieden sein können und Wasserstoff, eine Ethylgruppe oder eine N-Benzyl-N-methylaminoethylgruppe bedeuten, darstellt, an $R^{4''}$ monobromiert und mit entsprechend substituierten Alkoholen, Thiolen oder primären, sekundären und auch tertiären Aminen umsetzt und diese Produkte gegebenenfalls durch gezielte Oxidation in entsprechende Sulfinyl- oder Sulfonylverbindungen überführt,

oder

d) 1,6-Naphthyridin-Derivate der allgemeinen Formel Ib an $R^{4''}$ dibromiert und die gebildeten Dibrommethylverbindungen in andere funktionelle Gruppen wie Formyl oder Cyano überführt, oder

e) 1,6-Naphthyridinone-Derivate der allgemeinen Formel V, in denen $R^{5'}$ eine Cyanogruppe und $R^{4'}$ eine Methylgruppe bedeutet, welche man erhält, indem man in Analogie Zu bekannten Dihydropyridinsynthesen hergestellte Verbindungen der allgemeinen Formel Va

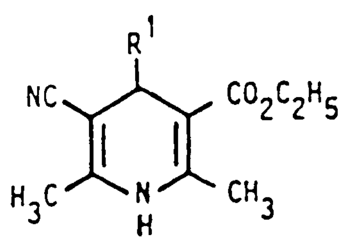

(Va)

in denen $R^1$ die oben genannte Bedeutung hat, mit s-Triazin umsetzt, in bekannter Weise alkyliert zu genannten 5-oxy-substituierten-1,6-Naphthyridinderivaten, die in 2-Methyl- und 3-cyano-substituiert sind.

Die für das Verfahren a) eingesetzten 1,6-Naphthyridinone der allgemeinen Formel V sind in P 33 27 650 beschrieben oder können in analoger Weise hergestellt werden.

Die Herstellung von oben genannten 1,6-Naphthyridin-Derivaten nach Verfahren a) erfolgt erfindungsgemäß nach üblichen, für die O-Alkylierung von Lactamen in der Literatur beschriebenen Verfahren (vgl. Adv. Heterocyclic Chem. 12 (1970), 185-212). Geeignete Alkylierungsmittel sind Alkylhalogenide und Alkylsulfonate, Dialkylsulfate und Trialkyloxoniumsalze.

Zur Reaktion mit Alkylhalogeniden werden die Verbindungen der allgemeinen Formel V in Form ihrer Metallsalze, vorzugsweise ihrer Alkali- oder Silbersalze, eingesetzt, die entweder separat hergestellt oder in situ mit Hilfe geeigneter Basen wie Metallhydriden, -carbonaten oder -alkoxiden in einem aprotischen Lösungsmittel erzeugt werden.

Als geeignete Lösungsmittel kommen, in Abhängigkeit vom jeweiligen Alkylierungsmittel, nahezu alle inerten organischen Lösungsmittel in Frage wie offenkettige, cyclische oder auch aromatische Kohlenwasserstoffe z.B. n-Pentan, n-Hexan, Cyclohexan, Benzol oder Toluol, halogenierte Kohlenwasserstoffe, wie Dichlormethan, 1,2-Dichlorethan, Ether wie z.B. Diethylether, 1,2-Dimethoxyethan, sowie dipolare aprotische Lösungsmittel wie Dimethylformamid, Dimethylacetamid, Hexamethylphosphoräuretriamid und Dimethylsulfoxid. Der Temperaturbereich kann je nach verwendetem Lösungsmittel zwischen -20°C und dem Siedepunkt des betreffenden Lösungsmittel variiert werden.

Aufgrund des ambidenten Charakters des Lactamanions erhält man bei der Alkylierung nach Verfahren a) vielfach Gemische aus O- und N-Alkylierungprodukten je nach Reaktionsbedingungen und verwendetem Alkylierungsmittel (J. Org. Chem. 32 (1967), 4040 ff). Die Trennung der erhaltenen Produktgemische kann durch chromatographische Methoden und/oder Kristallisation erfolgen.

Die oben genannten 1,6-Naphthyridin-Derivate, die in 5-Methoxy- oder 5-Ethoxy substituiert sind, werden bevorzugt durch Umsetzung der 1,6-Naphthyridinone der allgemeinen Formel V mit Trimethyl- bzw. Triethyloxoniumsalzen, insbesondere Trimethyloxonium- und Triethyloxoniumtetrafluorborat, in einem aprotischen Lösungsmittel erhalten. Die Herstellung der O-Propyl-, O-Isopropyl-, O-sec-Butyl- oder O-Benzylverbindungen erfolgt dagegen vorteilhaft durch Alkylierung der Alkalimetall- oder Silbersalze mit entsprechenden Alkyl- oder Benzylhalogeniden.

Die Herstellung der oben genannten Verbindungen nach Verfahren b) aus Verbindungen der allgemeinen Formel Ia, bei denen $R^5$ für einen Benzyloxycarbonylrest steht erfolgt erfindungsgemäß nach in der Literatur beschriebenen Verfahren (vgl. z.B. Houben-Weyl, Methoden der organischen Chemie, 4. Auflage, Bd. IV/1C, Reduktion Teil 1, 1980, S. 381-382).

Zur Hydrogenolyse der Benzylester wird vorzugsweise Palladium auf Aktivkohle als Katalysator eingesetzt. Als Lösungsmittel kommen vor allem niedere Alkohole, insbesondere Methanol oder Ethanol, in Frage. Die Temperatur kann zwischen 0 °C und dem Siedepunkt des betreffenden Lösungsmittels variiert werden, vorzugsweise wird bei Atmosphärendruck und einer Temperatur von 15 bis 30 °C gearbeitet. Zur Überführung der gebildeten Carbonsäure in ein Carbonsäurehalogenid, insbesondere ein Carbonsäurechlorid stehen verschiedene literaturbekannte Verfahren zur Auswahl. Zur Herstellung der Carbonsäurechloride eignet sich besonders das aus Oxalylchlorid und Dimethylformamid gebildete Dimethylchlormethylidenammoniumchlorid (vgl. Helv. Chim. Acta 61 (1978), 1675-1681) oder Phosphorpentachlorid (vgl. Chem. Pharm. Bull. 28 (1980), 2809-2812).

Bei der Umsetzung von Carbonsäuren mit Dimethylchlormethylidenammoniumchlorid wird unter Stickstoffatmosphäre in einem inerten organischen Lösungsmittel, wie z.B. Acetonitril oder Dioxan, und bei Temperaturen zwischen -30 °C und 30 °C, vorzugsweise jedoch bei 0 °C gearbeitet. Die anschießende Reaktion der Carbonsäurechloride mit entsprechend substituierten Alkoholen oder Aminen erfolgt im Eintopfverfahren bei Temperaturen von 0 °C bis 50 °C, vorzugsweise bei 20 °C.

Die Umsetzung der Carbonsäuren mit Phosphorpentachlorid wird in den in der Literatur für analoge Umsetzungen von Carbonsäuren beschriebenen Lösungsmitteln durchgeführt, vorzugsweise in chlorierten Kohlenwasserstoffen, wie Chloroform, Dichlormethan, 1,2-Dichlorethan oder Tetrachlorkohlenstoff im Temperaturbereich zwischen -20 °C und 20 °C.

Die Weiterreaktion zu Estern und Amiden erfolgt wie oben beschrieben.

Zur Herstellung der oben genannten Verbindungen nach Verfahren c) oder d), bei denen $R^4$ für eine substituierte Alkylgruppe steht, werden die entsprechenden 2-Methylanalogen mit Pyridiniumbromid-Perbromid in Gegenwart von Base an der Methylgruppe bromiert (vgl. Synthesis 1984, 617). Das Verhältnis von Mono- zu Dibromierungsprodukt wird dabei durch die Menge des eingesetzten Pyridiniumbromid Perbromids und durch die Reaktionsbedingungen gesteuert. Als Base wird Pyridin oder ein geeignetes tertiäres Amin eingesetzt. Da diese Basen je nach Reaktionsbedingungen teilweise selbst mit der Monobromverbindung zu Quartärsalzen reagieren und damit zu Nebenreaktionen führen können, wird bei den Umsetzungen, bei denen die Monobromverbindungen anschließend mit einem geeignet substituierten primären, sekundären oder tertiären Amin umgesetzt werden sollen, bereits dieses Amin als Hilfsbase bei der Bromierung eingesetzt.

Geeignete Lösungsmittel für die Bromierung sind insbesondere chlorierte Kohlenwasserstoffe, wie Chloroform, Dichlormethan, Tetrachlorkohlenstoff oder 1,2-Dichloräthan.

Zur Herstellung der monobromierten Verbindungen nach Verfahren c) wird bei Temperaturen zwischen -40 °C und 0 °C, vorzugsweise bei -20 °C, gearbeitet. Die monobromierten Verbindungen können im Eintopfverfahren mit Lösungen oder Suspensionen von mindestens drei Äquivalenten eines geeignet substituierten Alkoholates, Thiolates oder primären, sekundären oder tertiären Amins in einem inerten organischen Lösungsmittel umgesetzt werden. Dabei werden die Alkoholate bzw. Thiolate vorzugsweise aus dem entsprechenden Alkohol oder Thiol mit einer geeigneten Base, wie z.B. Natriumhydrid, in einem Lösungsmittel wie z.B. einem offenkettigen oder cyclischen Ether, vorzugsweise Tetrahydrofuran, in situ erzeugt und diese Lösung bzw. Suspension des Alkoholates bzw. Thiolates bei Temperaturen zwischen -40 °C und 30 °C mit der Brommethylverbindung umgesetzt.

Die Reaktionsprodukte der Brommethylverbindungen mit Thiolaten können mit ein bzw. zwei Äquivalenten einer Persäure, wie z.B. m-Chlorperbenzoesäure in einem geeigneten Lösungsmittel, wie z.B. chlorierten Kohlenwasserstoffen, vorzugsweise Dichlormethan, zu den entsprechenden Sulfoxiden bzw. Sulfonen oxidiert werden.

Zur Herstellung der Dibrommethylverbindungen nach Verfahren d) wird mit mindestens zwei Äquivalenten Pyridiniumbromid-Perbromid und bei Temperaturen zwischen -20 °C und 20 °C gearbeitet. Die Dibrommethylverbindungen werden durch Chromatographie und/oder Kristallisation isoliert und gegebenenfalls nach bekannten Verfahren in andere funktionelle Gruppen umgewandelt. So werden oben genannte Verbindungen bei denen $R^4$ eine Formylgruppe bedeutet, durch Hydrolyse der entsprechenden Dibrommethylverbindungen mit Silbernitrat, vorzugsweise in einem Gemisch aus Wasser und einem niederen Alkohol wie Methanol oder Ethanol, bei Temperaturen zwischen 20 °C und 100 °C hergestellt.

2-Cyano-Derivate werden aus 2-Formylderivaten nach bekannten Verfahren, vorzugsweise mit Hydroxylamin oder seinen Salzen in Gegenwart wasserabspaltender Mittel wie Acetanhydrid hergestellt (vgl. Sato J., Fujisawa Pharm. Co., Ltd., Belg. 879, 263). Dabei wird bei Temperaturen von 20 °C bis 150 °C, vorzugsweise bei 100 °C, in Eisessig gearbeitet.

Oben genannte saure oder basische Verbindungen, welche in 3-Position eine Carboxylgruppe bzw. ein basisches Zentrum an 2- oder 3-Position aufweisen, überführt man zum Zwecke der Reinigung und aus galenischen Gründen bevorzugt in kristalline, pharmakologisch verträgliche Salze.

Für den Fall, daß genannte Verbindungen in 3-Position durch substituiert sind, lassen sich mit Basen, wie z.B. Hydroxiden oder Carbonaten, entsprechende Salze der Alkali- oder Erdalkalimetalle herstellen. Wenn die Reste in 2- und/oder 3-Position basischen Charakter aufweisen, werden Salze in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Da die erfindungsgemäßen Verbindungen am C-4 ein chirales Zentrum aufweisen, können sie entweder als racemische Gemische oder in Form der Enantiomeren vorliegen.

Die Verbindungen sind hochwirksame Calciumantagonisten.

Aufgrund ihrer gefäßspasmolytischen Wirkungen sind sie vor allem bei cerebralen, cardialen und peripheren Gefäßerkrankungen indiziert. Die 1,6-Naphthyridin-Derivate der vorliegenden Erfindung sind daher wertvolle Mittel für die Bekämpfung der Herz-Kreislauf-Mortalität.

Die erfindungsgemäßen Verbindungen können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Ethylendiamintetraessigsäure und deren nichttoxische Salze) sowie hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykol); für orale Applikation geeignete Zubereitungen können gewünschtenfalls zusätzliche Geschmacks- und/oder Süßstoffe enthalten.

Die enteral verabreichten Einzeldosen liegen im Bereich von etwa 5 bis 250 mg, vorzugsweise 20 bis 100 mg. Parenteral werden etwa 1 bis 20 mg gegeben.

Die folgenden Beispiele dienen der näheren Erläuterung der Erfindung:

Beispiel 1:

(±)-1,4-Dihydro-5-isopropoxy-2-methyl-4-(2-thienyl)-1,6-naphthyridin-3-carbonsäureethylester

2,1 g (70 mMol) Natriumhydrid (80%ig in Öl) werden in 100 ml trockenem Dimethylformamid suspendiert und unter Rühren bei Raumtemperatur 20 g (63 mMol) (±)-1,4,5,6-Tetrahydro-2-methyl-5-oxo-4-(2-thienyl)-1,6-naphthyridin-3-carbonsäureethylester portionsweise fest zugegeben. Nach Abklingen der Gasentwicklung wird noch 30 Minuten bei Raumtemperatur nachgerührt. Dann werden 16 g (94 mMol) Isopropyljodid in 30 ml Dimethylformamid zugegeben. Man rührt 2 Tage bei Raumtemperatur, entfernt das Lösungsmittel im Vakuum und nimmt den Rückstand mit Wasser und Ethylacetat auf. Nach dem Ausschütteln wird die Ethylacetatphase abgetrennt und über Magnesiumsulfat getrocknet. Der nach Abdestillieren des Lösungsmittels erhaltene Rückstand wird chromatographisch an Kieselgel mit Toluol/Ethylacetat 3:1 getrennt. Die Fraktion mit dem Rf 0,3 wird isoliert und aus n-Hexan umkristallisiert.

Man erhält (±)-1,4-Dihydro-5-isopropoxy-2-methyl-4-(2-thienyl)-1,6-naphthyridin-3-carbonsäureethylester in Form farbloser Kristalle vom Schmp. 110-111 °C.

Der als Ausgangsprodukt verwendete (±)-1,4,5,6-Tetrahydro-2-methyl-5-oxo-4-(2-thienyl)-1,6-naphthyridin-3-carbonsäureethylester wird wie folgt hergestellt:
Zu einer Suspension von 6 g (0,2 Mol) Natriumhydrid (80%ig in Öl) in 100 ml trockenem Dimethylformamid wird unter Stickstoffatmosphäre eine Lösung von 67 g (0,2 Mol) 1,4-Dihydro-2,6-dimethyl-4-(2-thienyl)-pyridin-3,5-dicarbonsäurediethylester in 300 ml Dimethylformamid getropft. Bei Nachlassen der Gasentwicklung wird noch 10 Minuten bei Raumtemperatur gerührt und anschließend werden 16,2 g (0,2 Mol) s-Triazin in 300 ml Dimethylformamid zugetropft. Die Reaktionsmischung wird 16 Stunden auf 110 °C erhitzt und nach dem Abkühlen im Vakuum eingeengt. Der Rückstand wird an Kieselgel mit Dichlormethan/Methanol 9:1 chromatographiert. Die Fraktion mit dem Rf 0,4 wird isoliert und aus Ethanol umkristallisiert.

Man erhält (±)-1,4,5,6-Tetrahydro-2-methyl-5-oxo-4-(2-thienyl)-1,6-naphthyridin-3-carbonsäureethylester in Form hellbeiger Kristalle vom Schmp. 284-285 °C.

In analoger Weise werden die folgenden Verbindungen erhalten:

(±)-1,4-Dihydro-5-isopropoxy-2-methyl-4-(1-naphthyl)-1,6-naphthyridinon-3-carbonsäureethylester (1.a)
Schmp. 204-206 °C aus Diisopropylether/Ethanol

(±)-2-Amino-1,4-dihydro-5-isopropoxy-4-(2-trifluormethylphenyl)-1,6-naphthyridin-3-carbonsäureethylester (1.b)
Schmp. 173-175 °C aus n-Hexan/Diisopropylether

(±)-1,4-Dihydro-5-isopropoxy-2-methyl-4-(2-trifluormethylphenyl)-1,6-naphthyridin-3-carbonsäure-{2-[1-(4-diphenylmethylpiperazinyl)]ethyl}ester • 5 Oxalat (1.c)
Schmp.> 110 °C (Z) aus Ethylacetat/Isopropanol

Beispiel 2:

(±)-1,4-Dihydro-5-isopropoxy-2-[(4-methylpiperazin-1-yl)methyl]-4-phenyl-1,6-naphthyridin-3-carbonsäuremethylester

1,0 g (3,0 mMol) (±)-1,4-Dihydro-5-isopropoxy-2-methyl-4-phenyl-1,6-naphthyridin-3-carbonsäuremethylester (zur Herstellung vgl. P 34 31 303) in 20 ml ethanolfreiem Chloroform werden auf -10 °C gekühlt, mit 0,26 ml (3,2 mMol) Pyridin und anschließend portionsweise mit 1,1 g (3,1 mMol) Pyridiniumbromid Perbromid (90%ig) versetzt. Nach 30 Minuten bei -10 °C werden 0,62 g (6,2 mMol) N-Methylpiperazin zugetropft, 15 Minuten bei -10 °C gerührt, dann innerhalb einer Stunde auf 20 °C erwärmt. Es wird mit Chloroform verdünnt, mit Wasser gewaschen und die organische Phase über Natriumsulfat getrocknet. Der nach Abdestillieren des Lösungsmittels erhaltene Rückstand wird an Kieselgel mit Dichlormethan/Methanol (9:1) chromatographiert. Die Fraktion mit dem Rf 0,3 wird aus n-Hexan umkristallisiert.

Man erhält (±)-1,4-Dihydro-5-isopropoxy-2-[(4-methylpiperazin-1-yl)methyl]-4-phenyl-1,6-naphthyridin-3-carbonsäuremethylester in Form farbloser Kristalle vom Schmp. 149-151 °C.

In analoger Weise werden die folgenden Verbindungen erhalten:

(±)-2-Dimethylaminomethyl-1,4-dihydro-5-isopropoxy-4-(2-trifluormethylphenyl)-1,6-naphthyridin-3-carbonsäureethylester • Dihydrochlorid (2.a)
Schmp. 169-172 °C (Z) aus Ethylacetat/Isopropanol

(±)-[3-Ethoxycarbonyl-1,4-dihydro-5-isopropoxy-4-(2-trifluormethylphenyl)-1,6-naphthyridin-2-yl]methyl-1-pyridinumbromid (2.b)
Schmp. 206-207 °C aus Isopropanol

(±)-[3-Ethoxycarbonyl-1,4-dihydro-5-isopropoxy-4-(1-naphthyl)-1,6-naphthyridin-2-yl]methyl-(2-hydroxyethyl)-dimethylammoniumbromid (2.c)
Schmp: 212-215 °C aus Ethylacetat/Isopropanol

Beispiel 3:

(±)-2-(2-Aminoethoxymethyl)-4-(2-chlorphenyl)-1,4-dihydro-5-isopropoxy-1,6-naphthyridin-3-carbonsäureethylester • Dihydrochlorid

Eine Lösung von 5,0 g (12,9 mMol) (±)-4-(2-Chlorphenyl)-1,4-dihydro-5-isopropoxy-2-methyl-1,6-naphthyridin-3-carbonsäureethylester (zur Herstellung vgl. P 35 02 790) in 75 ml Dichlormethan wird auf -20 °C gekühlt, mit 1,1 ml (13,6 mMol) Pyridin und anschließend portionsweise mit 5,0 g (14,1 mMol) Pyridiniumbromid Perbromid (90%ig) versetzt. Es wird 1h bei -20 °C gerührt.

Parallel dazu wird eine Lösung von 2,6 g (42,6 mMol) 2-Aminoethanol in 100 ml Tetrahydrofuran bei 20 °C portionsweise mit 1,3 g (43,3 mMol) Natriumhydrid (80%ig in Öl) versetzt. Nach Beendigung der Wasserstoffentwicklung wird diese Lösung zu der obigen, auf -20 °C gekühlten Lösung des gebildeten (±)-2-Brommethyl-4-(2-chlorphenyl)-1,4-dihydro-5-isopropoxy-1,6-naphthyridin-3-carbonsäureethylesters getropft. Es wird auf Raumtemperatur erwärmt und 1h bei 20 °C gerührt. Nach Zugabe von 300 ml Wasser wird ausgeschüttelt, die organische Phase abgetrennt und über Natriumsulfat getrocknet. Der nach dem Abdestillieren der Lösungsmittel erhaltene Rückstand wird an Kieselgel mit Dichlormethan/methanol. Ammoniak (95:5, v/v) chromatographiert. Die Fraktion mit dem Rf 0,3 wird isoliert, in Ether/Ethylacetat gelöst und mit Chlorwasserstoff in Ether das Dihydrochlorid gefällt. Nach Umkristallisation aus Isopropanol erhält man (±)-2-(2-Aminoethoxymethyl)-4-(2-chlorphenyl)-1,4-dihydro-5-isopropoxy-1,6-naphthyridin-3-carbonsäureethylester • Dihydrochlorid in Form farbloser Kristalle vom Schmp. 187-188 °C.

In analoger Weise werden die folgenden Verbindungen erhalten:
(±)-2-(2-Aminoethoxymethyl)-1,4-dihydro-5-isopropoxy-4-(2-trifluormethylphenyl)-1,6-naphthyridin-3-carbonsäureethylester • Hydrochlorid (3.a)
Schmp. 200-203 ° C (Z) aus Acetonitril
(±)-1,4-Dihydro-5-isopropoxy-2-methoxmethyl-4-(2-trifluormethylphenyl)-1,6-naphthyridin-3-carbonsäureethylester (3.b)
Schmp. 161-162 ° C aus n-Hexan
(±)-1,4-Dihydro-2-hydroxymethyl-5-isopropoxy-4-(2-trifluormethylphenyl)-1,6-naphthyridin-3-carbonsäureethylester (3.c)
Schmp. 150-152 ° C aus n-Hexan/Diisopropylether
(±)-1,4-Dihydro-5-isopropoxy-2-phenylthiomethyl-4-(2-trifluormethylphenyl)-1,6-naphthyridin-3-carbonsäureethylester (3.d)
Schmp. 75-76 ° C aus n-Hexan
(±)-1,4-Dihydro-5-isopropoxy-2-(4-pyridylthiomethyl)-4-(2-trifluormethyl-phenyl)-1,6-naphthyridin-3-carbonsäureethylester (3.e)
Schmp. 214-216 ° C aus Ethylacetat
(±)-4-(2-Chlorphenyl)-1,4-dihydro-2-hydroxymethyl-5-isopropoxy-1,6-naphthyridin-3-carbonsäureethylester (3.f)
Schmp. 183-186 ° C aus n-Hexan/Diisopropylether
(±)-2-(2-N-Benzyl-N-methylaminoethoxymethyl)-1,4-dihydro-5-isopropoxy-4-(2-trifluormethylphenyl)-1,6-naphthyridin-3-carbonsäureethylester • Oxalat (3.g)
Schmp. 126-128 ° C aus Ethylacetat
(±)-2-(2-Dimethylaminoethoxymethyl)-1,4-dihydro-5-isopropoxy-4-(1-naphthyl)-1,6-naphthyridin-3-carbonsäureethylester (3.h)
Schmp. 134-137 ° C aus Diisopropylether/Ethylacetat

Beispiel 4:

(±)-2-Dibrommethyl-1,4-dihydro-5-isopropoxy-4-phenyl-1,6-naphthyridin-3-carbonsäureethylester

10 g (28,4 mMol) (±)-1,4-Dihydro-5-isopropoxy-2-methyl-4-phenyl-1,6-naphthyridin-3-carbonsäureethylester (zur Herstellung vgl. P 35 02 790) in 160 ml ethanolfreiem Chloroform werden auf 0 ° C gekühlt, mit 4,6 ml (56,8 mMol) Pyridin und anschließend portionsweise mit 20,2 g (56,8 mMol) Pyridiniumbromid Perbromid (90%ig) versetzt. Es wird 30 Minuten bei 0 ° C gerührt, dann 15 Minuten unter Rückfluß erhitzt. Nach dem Abkühlen wird mit Chloroform verdünnt, mit 2N Salzsäure, dann mit Wasser gewaschen und die organische Phase über Magnesiumsulfat getrocknet. Der nach dem Einengen erhaltene Rückstand wird an Kieselgel mit Dichlormethan/Methanol 9:1 chromatographiert. Als Fraktion mit dem Rf 0,7 wird der (±)-2-Dibrommethyl-1,4-dihydro-5-isopropoxy-4-phenyl-1,6-naphthyridin-3-carbonsäureethylester in Form eines hellgelben Öls isoliert.

In analoger Weise wird die folgende Verbindung erhalten:
(±)-4-(2-Bromophenyl)-2-dibrommethyl-1,4-dihydro-5-isopropoxy-1,6-naphthyridin-3-carbonsäuremethylester (4.a)
Schmp. 144-146 ° C aus n-Pentan

Beispiel 5:

(±)-1,4-Dihydro-5-isopropoxy-2-methyl-4-(2-trifluormethylphenyl)-1,6-naphthyridin-3-carbonsäure-[2-(N-benzyl-N-methylamino)ethyl]amid • Dihydrochlorid

17 ml trockenes Dimethylformamid in 40 ml trockenem Dioxan werden auf 0 ° C gekühlt und unter $N_2$-Atmosphäre 1,0 g (7,9 mMol) Oxalylchlorid zugetropft. Nach 20 min. bei 0 ° C werden portionsweise 2,7 g (6,9 mMol) (±)-1,4-Dihydro-5-isopropoxy-2-methyl-4-(2-trifluormethylphenyl)-1,6-naphthyridin-3-carbonsäure (EP 173 933) zugesetzt und auf 20 ° C erwärmt. Nach 30 min. bei 20 ° C wird diese Lösung zu einer auf 0 ° C gekühlten Lösung von 2,6 g (15,8 mMol) N-Benzyl-N-methylethylendiamin in 20 ml n-Hexan getropft. Nach Erwärmen auf 20 ° C wird 30 Minuten gerührt, dann unter erneuter Eiskühlung 2N Natriumcarbonatlösung zugetropft und die Reaktionsmischung zweimal mit Dichlormethan extrahiert. Die Dichlormethanlösungen werden mit Wasser gewaschen und über Natriumsulfat getrocknet. Das Lösungsmittel wird im Vakuum

entfernt und der Rückstand an Kieselgel mit Dichlormethan/Methanol (9:1, v/v) chromatographiert. Die Fraktion mit dem Rf 0,5 wird isoliert, in Ether gelöst und mit Chlorwasserstoff in Ether das Dihydrochlorid gefüllt. Dieses wird zweimal aus Acetonitril kristallisiert.

Man erhält (±)-1,4-Dihydro-5-isopropoxy-2-methyl-4-(2-trifluormethylphenyl)-1,6-naphthyridin-3-carbonsäure-[2-(N-benzyl-N-methylamino)ethyl]amid • Dihydrochlorid in Form farbloser Kristalle vom Schmp. 178-180 ° C.

In analoger Weise werden die folgenden Verbindungen erhalten:

(±)-1,4-Dihydro-5-isopropoxy-2-methyl-4-(2-trifluormethylphenyl)-1,6-naphthyridin-3-carbonsäure-N-ethylamid (5.a)
Schmp. 221-224 ° C aus Ethylacetat

(±)-1,4-Dihydro-5-isopropoxy-2-methyl-4-(2-trifluormethylphenyl)-1,6-naphthyridin-3-carbonsäureamid (5.b)
Schmp. 270-271 ° C aus Ethylacetat

Beispiel 6:

(±)-1,4-Dihydro-5-isopropoxy-2-phenylsulfinylmethyl-4-(2-trifluormethylphenyl)-1,6-naphthyridin-3-carbonsäureethylester

Eine Lösung von 1,0 g (1,9 mMol) (±)-1,4-Dihydro-5-isopropoxy-2-phenylthiomethyl-4-(2-trifluormethylphenyl)-1,6-naphthyridin-3-carbonsäureethylester (Beispiel 3.d) in 30 ml Dichlormethan wird auf 0 ° C gekühlt und 0,4 g (2,1 mMol) 3-Chlorperoxybenzoesäure (90%ig) portionsweise zugegeben. Nach 10 Minuten bei 0 ° C wurde Natriumhydrogencarbonatlösung zugetropft, die organische Phase abgetrennt, mit Wasser gewaschen und über Natriumsulfat getrocknet. Der nach Abdestillieren des Lösungsmittels erhaltene Rückstand wird aus Ethylacetat umkristallisiert.

Man erhält (±)-1,4-Dihydro-5-isopropoxy-2-phenylsulfinylmethyl-4-(2-trifluormethylphenyl)-1,6-naphthyridin-3-carbonsäureethylester in Form farbloser Kristalle vom Schmp. 206-208 ° C.

Beispiel 7:

(±)-1,4-Dihydro-5-isopropoxy-2-phenylsulfonylmethyl-4-(2-trifluormethylphenyl)-1,6-naphthyridin-3-carbonsäureethylester

Das bei der analog Beispiel 9 durchgeführten Umsetzung von (±)-1,4-Dihydro-5-isopropoxy-2-phenylthiomethyl-4-(2-trifluormethylphenyl)-1,6-naphthyridin-3-carbonsäureethylester (Beispiel 3.d) mit zwei Äquivalenten 3-Chlorperoxybenzoesäure und einer Reaktionsdauer von 16 Stunden bei 20 ° C erhaltene Rohprodukt wird chromatographisch an Kieselgel mit Toluol/Ethylacetat 3:1 getrennt. Die Fraktion mit dem Rf 0,3 wird isoliert und aus Diisopropylether umkristallisiert.

Man erhält (±)-1,4-Dihydro-5-isopropoxy-2-phenylsulfonylmethyl-4-(2-trifluormethylphenyl)-1,6-naphthyridin-3-carbonsäureethylester in Form farbloser Kristalle vom Schmp. 177-182 ° C.

Beispiel 8 :

(±)-1,4-Dihydro-5-isopropoxy-2-methyl-4-(2-trifluormethylphenyl)-1,6-naphthyridin-3-carbonsäure-(2-aminoethyl)ester

15,0 g (24,4 mMol) (±)-1,4-Dihydro-5-isopropoxy-2-methyl-4-(2-trifluormethylphenyl)-1,6-naphthyridin-3-carbonsäure-[2-(N,N-dibenzylamino)ethyl]ester (Base von Beispiel 4 ) werden mit 5,0 g Palladium auf Aktivkohle (10%) in 300 ml Ethanol bei Normaldruck und Raumtemperatur hydriert. Nach beendeter Wasserstoffaufnahme wird vom Katalysator abfiltriert, im Vakuum eingeengt und der Rückstand aus Diisopropylether/Ethylacetat umkristallisiert.

Man erhält (±)-1,4-Dihydro-5-isopropoxy-2-methyl-4-(2-trifluormethylphenyl)-1,6-naphthyridin-3-carbonsäure-(2-aminoethyl)ester in Form farbloser Kristalle vom Schmp. 166-167 ° C.

In analoger Weise wird die folgende Verbindung erhalten:

(±)-2-Formyl-1,4-dihydro-5-isopropoxy-4-(2-trifluormethylphenyl)-1,6-naphthyridin-3-carbonsäureethylester (8.a)
Schmp. 108-109 ° C aus n-Hexan

Beispiel 9:

(±)-2-Cyano-1,4-dihydro-5-isopropoxy-4-(2-trifluormethylphenyl)-1,6-naphthyridin-3-carbonsäureethylester

Eine Lösung von 1,00 g (2,30 mMol) (±)-2-Formyl-1,4-dihydro-5-isopropoxy-4-(2-trifluormethylphenyl)-1,6-naphthyridin-3-carbonsäureethylester (Beispiel 8.a) in 15 ml Eisessig wird mit 0,22 g (3,17 mMol) Hydroxylamin Hydrochlorid und 0,29 g (3,54 mMol) wasserfreiem Natriumacetat 2,5 Stunden bei 20°C gerührt. Es werden 0,80 ml (8,46 mMol) Acetanhydrid zugegeben und 1,5 Stunden bei 20°C, dann 10 Stunden bei 100°C gerührt. Der nach Einengen im Vakuum erhaltene ölige Rückstand wird mit 50 ml Wasser versetzt, mit gesättigter Kaliumhydrogencarbonatlösung neutralisiert und zweimal mit je 30 ml Dichlormethan extrahiert. Die Dichlormethanlösung wird über Natriumsulfat getrocknet, filtriert und der nach Abdestillieren des Lösungsmittels erhaltene Rückstand am Kieselgel mit Toluol/Ethylacetat 9 : 1 chromatographiert. Die Fraktion mit dem Rf 0,2 wird isoliert und aus n-Hexan umkristallisiert.

Man erhält (±)-2-Cyano-1,4-dihydro-5-isopropoxy-4-(2-trifluormethylphenyl)-1,6-naphthyridin-3-carbonsäureethylester in Form blaßgelber Kristalle vom Schmp. 138-140°C.

Beispiel 10:

(±)-1,4-Dihydro-5-isopropoxy-2-methyl-4-(2-trifluormethylphenyl)-1,6-naphthyridin-3-carbonitril

8,0 g (24,1 mMol) (±)-1,4,5,6-Tetrahydro-2-methyl-5-oxo-4-(2-trifluormethylphenyl)-1,6-naphthyridin-3-carbonitril werden mit 5,0 g (50,0 mMol) Calciumcarbonat und 12,5 g (73,5 mMol) Isopropyljodid in 100 ml Dimethylacetamid 10 Stunden bei 100°C erhitzt. Nach dem Abkühlen wird im Vakuum eingedampft. Der Rückstand wird mit 100 ml Wasser und 100 ml Dichlormethan ausgeschüttelt. Die organische Phase wird abgetrennt, über Natriumsulfat getrocknet und der nach Abdestillieren des Lösungsmittels erhaltene Rückstand an Kieselgel mit Toluol/Ethylacetat 1 : 1 chromatographiert. Die Fraktion mit dem Rf 0,35 wird isoliert und aus Diisopropylether/Ethylacetat umkristallisiert.

Man erhält (±)-1,4-Dihydro-5-isopropoxy-2-methyl-4-(2-trifluormethylphenyl)-1,6-naphthyridin-3-carbonitril in Form farbloser Kristalle vom Schmp. 207-209°C.

Das als Ausgangsprodukt verwendete (±)-1,4,5,6-Tetrahydro-2-methyl-5-oxo-4-(2-trifluormethylphenyl)-1,6-naphthyridin-3-carbonitril wird wie folgt hergestellt:
Zu einer Suspension von 4,5 g (0,15 Mol) Natriumhydrid (80%ig in Öl) in 50 ml trockenem Dimethylformamid wird unter Stickstoffatmosphäre eine Lösung von 43,7 g (0,12 Mol) (±)-5-Cyano-1,4-dihydro-2,6-dimethyl-4-(2-trifluormethylphenyl)pyridin-3-carbonsäureethylester in 150 ml Dimethylformamid getropft. Bei Nachlassen der Gasentwicklung wird noch 10 Minuten bei Raumtemperatur gerührt und anschließend werden 10,1 g (0,12 Mol) s-Triazin in 200 ml Dimethylformamid zugetropft. Die Reaktionsmischung wird 16 Stunden auf 100°C erhitzt und nach dem Abkühlen im Vakuum eingeengt. Der Rückstand wird an Kieselgel mit Dichlormethan/Methanol 9 : 1 chromatographiert. Die Fraktion mit dem Rf 0,4 wird isoliert und aus Ethanol umkristallisiert.

Man erhält (±)-1,4,5,6-Tetrahydro-2-methyl-5-oxo-4-(2-trifluormethylphenyl)-1,6-naphthyridin-3-carbonitril in Form beiger Kristalle, die sich oberhalb 290°C zersetzen.

Der als Ausgangsmaterial verwendete (±)-5-Cyano-1,4-dihydro-2,6-dimethyl-4-(2-trifluormethylphenyl)-pyridin-3-carbonsäureethylester wird in an sich bekannter Weise analog anderen Dihydropyridinsynthesen aus 2'-Trifluormethylbenzylidenacetessigsäureethylester und 3-Aminocrotonsäurenitril durch fünfstündiges Sieden in Isopropanol hergestellt.

Beispiel 11:

(±)-1,4-Dihydro-5-(1-dimethylamino-2-propoxy)-2-methyl-4-(2-trifluormethylphenyl)-1,6-naphthyridin-3-carbonsäureethylester • Dihydrochlorid

20 g (53 mMol) (±(-1,4,5,6-Tetrahydro-2-methyl-5-oxo-4-(2-trifluormethylphenyl)-1,6-naphthyridin-3-carbonsäureethylester (P 33 27 650, Beispiel s), 10,6 g (106 mMol) Calciumcarbonat, 20 g (160 mMol) 1-Dimethylamino-2-propylchlorid und eine katalytische Menge Kaliumjodid werden in 200 ml Dimethylacetamid 16 Stunden bei 100°C gerührt. Nach dem Abkühlen wird filtriert und in Vakuum eingedampft. Der Rückstand wird mit 100 ml in Natronlauge und 100 ml Dichlormethan ausgeschüttelt. Die organische Phase wird abgetrennt, mit Wasser gewaschen, über Natriumsulfat getrocknet und eingedampft. Das Rohprodukt

wird an Kieselgel mit Dichlormethan/Methanol 9 : 1 chromatographiert und die Fraktion mit dem Rf 0,2 isoliert. Diese wird in 100 ml Ether gelöst und mit Chlorwasserstoff in Ether das Dihydrochlorid gefällt, abfiltriert und aus Ethylacetat/Ethanol umkristallisiert.

Man erhält (±)-1,4-Dihydro-5-(1-dimethylamino-2-propoxy)-2-methyl-4-(2-trifluormethylphenyl)-1,6-naphthyridin-3-carbonsäureethylester • Dihydrochlorid in Form blaßbeiger Kristalle vom Schmp. 153-156° C.

Beispiel 12:

(±)-1,4-Dihydro-2-(2-hydroxyethoxymethyl)-5-isopropoxy-4-(2-trifluormethylphenyl)-1,6-naphthyridin-3-carbonsäureethylester

2,75 g (4,82 mMol) (±)-2-(2-Benzyloxyethoxymethyl)-1,4-dihydro-5-isoproxoxy-4-(2-trifluormethylphenyl)-1,6-naphthyridin-3-carbonsäureethylester (Beispiel 3.i) werden mit 1,3 g Palladium auf Aktivkohle (10 %) in 250 ml Ethanol bei Normaldruck und 50° C hydriert. Nach beendeter Wasserstoffaufnahme wird vom Katalysator abfiltriert, in Vakuum eingeengt und der Rückstand aus n-Hexan umkristallisiert.

Man erhält (±)-1,4-Dihydro-2-(2-hydroxyethoxymethyl)-5-isopropoxy-4-(2-trifluormethylphenyl)-1,6-naphthyridin-3-carbonsäureethylester in Form farbloser Kristalle vom Schmp. 109-112° C.

## Patentansprüche

**1.** 5-Oxy-substituierte -1,6-Naphthyridin-Derivate, nämlich

(±)-2-Amino-1,4-dihydro-5-isopropoxy-4-(2-trifluormethylphenyl)-1,6-napthyridin-3-carbonsäureethylester,

(±)-1,4-Dihydro-5-isopropoxy-2-methyl-4-(2-trifluormethylphenyl)-1,6-naphthyridin-3-carbonsäure-{2-[1-(4-diphenylmethylpiperazinyl)]ethyl}ester • 5 Oxalat,

(±)-2-Dimethylaminomethyl-1,4-dihydro-5-isopropoxy-4-(2-trifluormethylphenyl)-1,6-naphthyridin-3-carbonsäureethylester • Dihydrochlorid,

(±)-[3-Ethoxycarbonyl-1,4-dihydro-5-isopropoxy-4-(2-trifluormethylphenyl)-1,6-naphthyridin-2-yl]methyl-1-pyridiniumbromid,

(±)-2-(2-Aminoethoxymethyl)-1,4-dihydro-5-isopropoxy-4-(2-trifluormethylphenyl)-1,6-naphthyridin-3-carbonsäureethylester • Hydrochlorid,

(±)-1,4-Dihydro-5-isopropoxy-2-methoxymethyl-4-(2-trifluormethylphenyl)-1,6-naphthyridin-3-carbonsäureethylester,

(±)-1,4-Dihydro-2-hydroxymethyl-5-isopropoxy-4-(2-trifluormethylphenyl)-1,6-naphthyridin-3-carbonsäureethylester,

(±)-1,4-Dihydro-5-isopropoxy-2-phenylthiomethyl-4-(2-trifluormethylphenyl)-1,6-naphthyridin-3-carbonsäureethylester,

(±)-1,4-Dihydro-5-isopropoxy-2-(4-pyridylthiomethyl)-4-(2-trifluormethylphenyl)-1,6-naphthyridin-3-carbonsäureethylester,

(±)-2-(2-N-Benzyl-N-methylaminoethoxymethyl)-1,4-dihydro-5-isopropoxy-4-(2-trifluormethylphenyl)-1,6-naphthyridin-3-carbonsäureethylester • 2 Oxalat,

(±)-1,4-Dihydro-5-isopropoxy-2-methyl-4-(2-trifluormethylphenyl)-1,6-naphthyridin-3-carbonsäure-[2-(N-benzyl-N-methylamino)ethyl]-amid • Dihydrochlorid,

(±)-1,4-Dihydro-5-isopropoxy-2-methyl-4-(2-trifluormethylphenyl)-1,6-naphthyridin-3-carbonsäure-N-ethylamid,

(±)-1,4-Dihydro-5-isopropoxy-2-methyl-4-(2-trifluormethylphenyl)-1,6-naphthyridin-3-carbonsäureamid,

(±)-1,4-Dihydro-5-isopropoxy-2-phenylsulfinylmethyl-4-(2-trifluormethylphenyl)-1,6-naphthyridin-3-carbonsäureethylester,

(±)-1,4-Dihydro-5-isopropoxy-2-phenylsulfonylmethyl-4-(2-trifluormethylphenyl)-1,6-naphthyridin-3-carbonsäureethylester,

(±)-1,4-Dihydro-5-isopropoxy-2-methyl-4-(2-trifluormethylphenyl)-1,6-naphthyridin-3-carbonsäure-(2-aminoethyl)ester,

(±)-2-Formyl-1,4-dihydro-5-isopropoxy-4-(2-trifluormethylphenyl)-1,6-naphthyridin-3-carbonsäureethylester,

(±)-2-Cyano-1,4-dihydro-5-isopropoxy-4-(2-triflourmethylphenyl)-1,6-naphthyridin-3-carbonsäureethylester,

(±)-1,4-Dihydro-5-isopropoxy-2-methyl-4-(2-triflourmethylphenyl)-1,6-naphthyridin-3-carbonitril,

(±)-1,4-Dihydro-5-(1-dimethylamino-2-propoxy)-2-methyl-4-(2-trifluormethylphenyl)-1,6-naphthyridin-3-carbonsäureethylester • Dihydrochlorid und

(±)-1,4-Dihydro-2-(2-hydroxyethoxymethyl)-5-isopropoxy-4-(2-trifluormethylphenyl)-1,6-naphthyridin-3-carbonsäureethylester,

**2.** 5-oxy-substituierte-1,6-Naphthyridin-Derivate, nämlich

(±)-2-(2-Dimethylaminoethoxymethyl)-1,4-dihydro-5-isopropoxy-4-(1-naphthyl)-1,6-naphthyridin-3-carbonsäureethylester,

(±)-1,4-Dihydro-5-isopropoxy-2-methyl-4-(1-naphthyl)-1,6-naphthyridin-3-carbonsäureethylester und

(±)-[3-Ethoxycarbonyl-1,4-dihydro-5-isopropoxy-4-(1-naphthyl)-1,6-naphthyridin-2-yl]methyl-(2-hydroxyethyl)-dimethylammoniumbromid.

**3.** 5-oxy-substituierte-1,6-Naphthyridin-Derivate nämlich

(±)-2-Dibrommethyl-1,4-dihydro-5-isopropoxy-4-phenyl-1,6-naphthyridin-3-carbonsäureethylester und

(±)-1,4-Dihydro-5-isopropoxy-2-[(4-methylpiperazin-1-yl)methyl]-4-phenyl-1,6-naphthyridin-3-carbonsäuremethylester.

**4.** 5-oxy-substituierte-1,6-Naphthyridin-Derivate, nämlich

(±)-4-(2-Chlorphenyl)-1,4-dihydro-2-hydroxymethyl-5-isopropoxy-1,6-naphthyridin-3-carbonsäureethylester und

(±)-2-(2-Aminoethoxymethyl)-4-(2-chlorphenyl)-1,4-dihydro-5-isopropoxy-1,6-naphthyridin-3-carbonsäureethylester • Dihydrochlorid.

**5.** (±)-4-(2-Bromphenyl)-2-dibrommethyl-1,4-dihydro-5-isopropoxy-1,6-naphthyridin-3-carbonsäuremethylester.

**6.** Verfahren zur Herstellung der in den Ansprüchen 1 bis 5 genannten 5-oxy- substituierten 1,6-Naphthyridin-Derivate, dadurch gekennzeichnet, daß man entweder

a) 1,6-Naphthyridinon-Derivate der allgemeinen Formel V

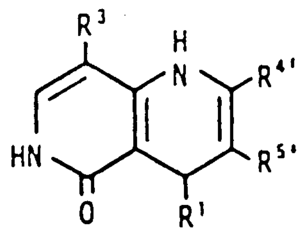

(V)

in welcher $R^1$ und $R^3$ die gemäß der genannten Verbindungen entsprechende Bedeutung haben, $R^{4'}$ eine Methyl- oder Aminogruppe und $R^{5'}$ einen Methoxycarbonyl-, Ethoxycarbonyl- oder oder N-Diphenylmethylpiperazinylethoxycarbonylrest oder einen Carboxamidrest der allgemeinen Formel IV

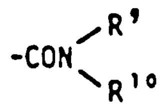

(IV)

worin $R^9$ und $R^{10}$ gleich oder verschieden sein können und Wasserstoff, eine Ethylgruppe oder eine N-Benzyl-N-methylaminoethylgruppe darstellen, bedeutet, in an sich bekannter Weise am Lactamsauerstoffatom alkyliert oder

b) 1,6-Naphthyridin-Derivate der allgemeinen Formel Ia

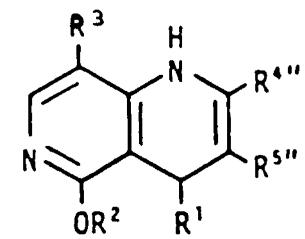

(Ia)

in welcher $R^1$ und $R^3$ die oben angegebene Bedeutung haben, $R^2$ für eine geradkettige oder verzweigte Alkylgruppe mit bis zu 4 Kohlenstoffatomen steht $R^{4''}$ für eine Methylgruppe und $R^{5''}$ für einen Benzyloxycarbonylrest steht, in an sich bekannter Weise hydrogenolytisch zu entsprechenden Carbonsäuren spaltet und diese wiederum in an sich bekannter Weise über die Zwischenstufe eines Säurehalogenides mit entsprechend substituierten Alkoholen oder Aminen zu Ester- bzw. Amidderivaten der 1,6-Naphthyridine der allgemeinen Formel I umsetzt,
oder

c) 1,6-Naphthyridin-Derivate der allgemeinen Formel Ib,

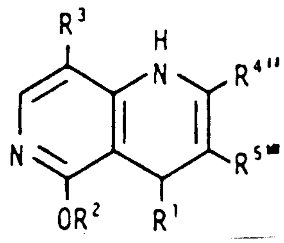

(Ib)

in welcher $R^1$, $R^2$, $R^3$ und $R^4$ die oben angegebene Bedeutung haben und $R^{5'''}$, einen Methoxycarbonyl-, Ethoxycarbonyl- oder N-Diphenylmethylpiperazinylethoxycarbonylrest oder einen Carboxamidrest der allgemeinen Formel IV

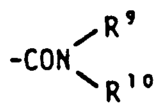

(IV)

worin $R^9$ und $R^{10}$ gleich oder verschieden sein können und Wasserstoff, eine Ethylgruppe oder eine N-Benzyl-N-methylaminoethylgruppe bedeuten, darstellt, an $R^{4''}$ monobromiert und mit entsprechend substituierten Alkoholen, Thiolen oder primären, sekundären und auch tertiären Aminen umsetzt und diese Produkte gegebenenfalls durch gezielte Oxidation in entsprechende Sulfinyl- oder Sulfonylverbindungen überführt,
oder
d) 1,6-Naphthyridin-Derivate der allgemeinen Formel Ib an $R^{4''}$ dibromiert und die gebildeten Dibrommethylverbindungen in andere funktionelle Gruppen wie Formyl oder Cyano überführt, oder
e) 1,6-Naphthyridinone-Derivate der allgemeinen Formel V, in denen $R^{5'}$ eine Cyanogruppe und $R^{4'}$ eine Methylgruppe bedeutet, welche man erhält, indem man in Analogie zu bekannten Dihydropyridinsynthesen hergestellte Verbindungen der allgemeinen Formel Va

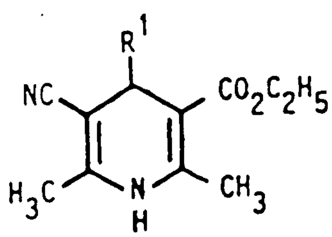

(Va)

in denen $R^1$ die oben genannte Bedeutung hat, mit s-Triazin umsetzt, in bekannter Weise alkyliert zu Verbindungen der allgemeinen Formel I, in denen $R^5$ eine Cyanogruppe und $R^4$ eine Methylgruppe bedeutet
f) 1,6-Naphthyridin-Derivate der allgemeinen Formel I, in der $R^5$ für einen N,N-Dibenzylaminoalkoxycarbonylrest steht in an sich bekannter Weise hydrogenolytisch spaltet Zu Verbindungen der allgemeinen Formel I, bei der $R^5$ für eine unsubstituierte Aminoalkoxycarbonyl- oder Aminoalkylcarbamoylgruppe steht, oder
g) 1,6-Naphthyridin-Derivate der allgemeinen Formel I, bei denen $R^4$ für einen N-Benzylaminoalkoxymethyl-, einen N,N-Dibenzylaminoalkoxymethyl- oder einen Azidoalkoxymethylrest steht, in an sich bekannter Weise hydrogenolytisch spaltet zu Verbindungen der allgemeinen Formel I, bei denen $R^4$ für einen unsubstituierten Aminoalkoxymethylrest steht.

**7.** Arzneimittel, dadurch gekennzeichnet, daß sie neben üblichen Hilfs- und Zusatzstoffen Verbindungen der Ansprüche 1 bis 5 enthalten, zur Behandlung von Gefäßerkrankungen.

8. Verfahren zur Verwendung von Verbindungen gemäß der Ansprüche 1 bis 5 zur Herstellung von Arzneimitteln zur Behandlung von Gefäßerkrankungen.

**Claims**

1. 5-Oxy-substituted-1,6-naphthyridine-derivatives, namely

(±)-2-amino-1,4-dihydro-5-isopropoxy-4-(2-trifluoromethylphenyl)-1,6-naphthyridine-3-carboxylic acid ethyl ester,

(±)-1,4-dihydro-5-isopropoxy-2-methyl-4-(2-trifluoromethylphenyl)-1,6-naphthyridine-3-carboxylic acid-{2-[1-(4-diphenylmethylpiperazinyl)]ethyl}ester • 5 oxalate,

(±)-2-dimethylaminomethyl-1,4-dihydro-5-isopropoxy-4-(2-trifluoromethylphenyl)-1,6-naphthyridine-3-carboxylic acid ethyl ester • dihydrochloride,

(±)-[3-ethoxycarbonyl-1,4-dihydro-5-isopropoxy-4-(2-trifluoromethylphenyl)-1,6-naphthyridine-2-yl]-methyl-1-pyridinium bromide,

(±)-2-(2-aminoethoxymethyl)-1,4-dihydro-5-isopropoxy-4-(2-trifluoromethylphenyl)-1,6-naphthyridine-3-carboxylic acid ethyl ester • hydrochloride,

(±)-1,4-dihydro-5-isopropoxy-2-methoxymethyl-4-(2-trifluoromethylphenyl)-1,6-naphthyridine-3-carboxylic acid ethyl ester,

(±)-1,4-dihydro-2-hydroxymethyl-5-isopropoxy-4-(2-trifluoromethylphenyl)-1,6-naphthyridine-3-carboxylic acid ethyl ester,

(±)-1,4-dihydro-5-isopropoxy-2-phenylthiomethyl-4-(2-trifluoromethylphenyl)-1,6-naphthyridine-3-carboxylic acid ethyl ester,

(±)-1,4-dihydro-5-isopropoxy-2-(4-pyridylthiomethyl)-4-(2-trifluoromethylphenyl)-1,6-naphthyridine-3-carboxylic acid ethyl ester,

(±)-2-(2-N-benzyl-N-methylaminoethoxymethyl)-1,4-dihydro-5-isopropoxy-4-(2-trifluoromethylphenyl)-1,6-naphthyridine-3-carboxylic acid ethyl ester • 2 oxalate,

(±)-1,4-dihydro-5-isopropoxy-2-methyl-4-(2-trifluoromethylphenyl)-1,6-naphthyridine-3-carboxylic acid-[2-(N-benzyl-N-methylamino)ethyl]amide • dihydrochloride,

(±)-1,4-dihydro-5-isopropoxy-2-methyl-4-(2-trifluoromethylphenyl)-1,6-naphthyridine-3-carboxylic acid-N-ethylamide,

(±)-1,4-dihydro-5-isopropoxy-2-methyl-4-(2-trifluoromethylphenyl)-1,6-naphthyridine-3-carboxylic acid amide,

(±)-1,4-dihydro-5-isopropoxy-2-phenylsulphinylmethyl-4-(2-trifluoromethylphenyl)-1,6-naphthyridine-3-carboxylic acid ethyl ester,

(±)-1,4-dihydro-5-isopropoxy-2-phenylsulphonylmethyl-4-(2-trifluoromethylphenyl)-1,6-naphthyridine-3-carboxylic acid ethyl ester,

(±)-1,4-dihydro-5-isopropoxy-2-methyl-4-(2-trifluoromethylphenyl)-1,6-naphthyridine-3-carboxylic acid-(2-aminoethyl)ester,

(±)-2-formyl-1,4-dihydro-5-isopropoxy-4-(2-trifluoromethylphenyl)-1,6-naphthyridine-3-carboxylic acid ethyl ester,

(±)-2-cyano-1,4-dihydro-5-isopropoxy-4-(2-trifluoromethylphenyl)-1,6-naphthyridine-3-carboxylic acid ethyl ester,

(±)-1,4-dihydro-5-isopropoxy-2-methyl-4-(2-trifluoromethylphenyl)-1,6-naphthyridine-3-carbonitrile,

(±)-1,4-dihydro-5-(1-dimethylamino-2-propoxy)-2-methyl-4-(2-trifluoromethylphenyl)-1,6-naphthyridine-3-carboxylic acid ethyl ester • dihydrochloride and

(±)-1,4-dihydro-2-(2-hydroxyethoxymethyl)-5-isopropoxy-4-(2-trifluoromethylphenyl)-1,6-naphthyridine-3-carboxylic acid ethyl ester.

**2.** 5-Oxy-substituted-1,6-naphthyridine-derivatives, namely

(±)-2-(2-dimethylaminoethoxymethyl)-1,4-dihydro-5-isopropoxy-4-(1-naphthyl)-1,6-naphthyridine-3-carboxylic acid ethyl ester,

(±)-1,4-dihydro-5-isopropoxy-2-methyl-4-(1-naphthyl)-1,6-napthyridine-3-carboxylic acid ethyl ester and

(±)-[3-ethoxycarbonyl-1,4-dihydro-5-isopropoxy-4-(1-naphthyl)-1,6-naphthyridine-2-yl]methyl-(2-hydroxyethyl)-dimethylammonium bromide.

**3.** 5-Oxy-substituted-1,6-naphthyridine-derivatives namely

(±)-2-dibromomethyl-1,4-dihydro-5-isopropoxy-4-phenyl-1,6-naphthyridine-3-carboxylic acid ethyl ester,

(±)-1,4-dihydro-5-isopropoxy-2-[(4-methylpiperazine-1-yl)methyl]-4-phenyl-1,6-naphthyridine-3-carboxylic acid ethyl ester.

**4.** 5-Oxy-substituted-1,6-naphthyridine-derivatives, namely

(±)-4-(2-chlorophenyl)-1,4-dihydro-2 -hydroxymethyl-5-isopropoxyl-1,6-naphthyridine-3-carboxylic acid ethyl ester,

(±)-2-(2-aminoethoxymethyl)-4-(2-chlorophenyl)-1, 4-dihydro-5-isopropoxyl-1,6-naphthyridine-3-carboxylic acid ethyl ester • dihydrochloride.

**5.** (±)-4-(2-Bromophenyl)-2-dibromomethyl-1,4- dihydro-5-isopropoxy-1,6-napthyridine-3-carboxylic acid ethyl ester.

**6.** A process for the preparation of the 5-oxy-substituted 1,6-naphthyridine derivatives according to claims 1 to 5 characterised in that either

a) there are alkylated in a known manner on the lactam oxygen atom, 1,6-naphthyridinone derivatives of the general formula V

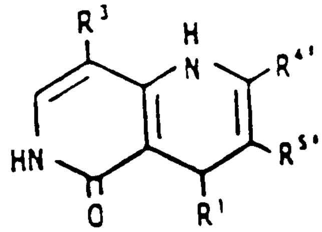

(V)

in which $R^1$ and $R^3$ have in accordance with the named compounds the corresponding meaning, $R^{4'}$ signifies a methyl or amino group and $R^{5'}$ a methoxycarbonyl-, ethoxycarbonyl- or N-diphenylmethylpiperazinylethoxycarbonyl radical or a carboxamide radical of the general formula IV

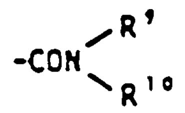

(IV)

wherein $R^9$ and $R^{10}$ can be the same or different and represent a hydrogen, an ethyl group or an N-benzyl-N-methylaminoethyl,

or

b) there are split hydrogenolytically in a known manner to corresponding carboxylic acids, 1,6-naphthyridine derivatives of the general formula Ia

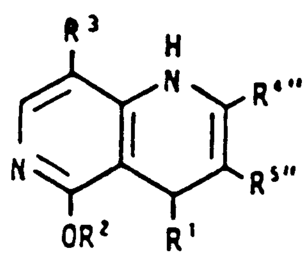

(Ia)

in which $R^1$ and $R^3$ have the above-given meaning, $R^2$ represents a straight-chained or branched alkyl group with up to four carbon atoms, $R^{4''}$ represents a methyl group, and $R^{5''}$ for a benzyloxycarbonyl radical, and these, in turn, are reacted in a known manner, by way of the intermediate stage of a halic acid with correspondingly substituted alcohols or amines, to esters or amide derivatives of the 1,6-naphthyridine derivatives of the general formula I,

or

c) there are monobrominated at $R^{4''}$, 1,6-naphthyridine derivatives of the general formula Ib,

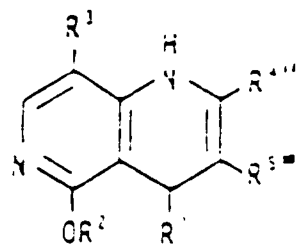

(Ib)

in which $R^1$, $R^2$, $R^3$ and $R^{4''}$ have the above-given meaning and $R^{5'''}$ represents a methoxycarbonyl-, ethoxycarbonyl- or N-diphenylmethylpiperazinylethoxycarbonyl radical or a carboxamide radical of the general formula IV

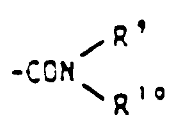

(IV)

wherein $R^9$ and $R^{10}$ can be the same or different and signify hydrogen, an ethyl group or a N-benzyl-N-methylaminoethyl group; and [these are] reacted with correspondingly substituted alcohols,

thiols or primary, secondary and also tertiary amines, and these products are possibly converted by directed oxidation into corresponding sulphinyl or sulphonyl compounds,

or
d) there are dibrominated at $R^{4''}$, 1,6-naphythyridine derivatives of the general formula Ib and the dibromomethyl compounds formed are converted into other functional groups such as formyl or cyano,

or
e) there are obtained, 1,6-naphthyridinone derivatives of the general formula V, in which $R^{5'}$ signifies a cyano group and $R^{4'}$ a methyl group, in that compounds of the general formula Va, produced in an analogous manner to the known dihydropyridine synthesis,

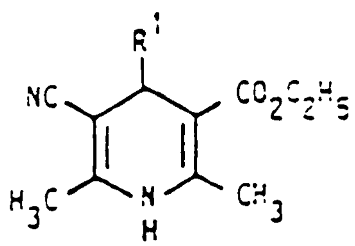
(Va)

in which $R^1$ has the above meaning, are reacted with s-triazine, alkylated in a known manner to [form] compounds of the general formula I, in which $R^5$ signifies a cyano group and $R^4$ a methyl group,
f) there are split hydrogenolytically in a known manner, 1,6-naphthyridine derivatives of the general formula I, in which $R^5$ represents a N,N-dibenzylaminoalkoxycarbonyl radical, to [form] compounds of the general formula I, in which $R^5$ represents an unsubstituted aminoalkoxylcarbonyl- or aminoalkylcarbamoyl group or
g) there are split hydrogenolytically in a known manner, 1,6-naphthyridine derivatives of the general formula I, in which $R^4$ represents an N-benzylaminoalkoxymethyl-, an N,N-dibenzylaminoalkoxymethyl or an azidoalkoxymethyl radical, to [form] compounds of the general formula I, in which $R^4$ represents an unsubstituted aminoalkoxymethyl radical.

**7.** Medicine, characterized in that, as well as auxiliary and added substances, it contains compounds of claims 1 to 5, for treating blood vessel diseases.

**8.** A method for using compounds, according to claims 1 to 5 for preparing medicine for treating blood vessel diseases.

## Revendications

**1.** Dérivés 5-oxy-substitués de 1,6-naphthyridine, à savoir :
l'éthylester d'acide (±)-2-amino-1,4-dihydro-5-isopropoxy-4-(2-trifluorométhylphényl)-1,6-naphthyridine-3-carboxylique,
le 5-oxalate de (2-[1-(4-diphénylméthylpipérazinyl)-ester d'acide (±)-1,4-dihydro-5-isopropoxy-2-méthyl-4-(2-trifluorométhylphényl)-1,6-naphthyridine-3-carboxylique,
le dichlorhydrate de l'éthylester d'acide (±)-2-diméthylaminométhyl-1,4-dihydro-5-isopropoxy-4-(2-trifluorométhylphényl)-1,6-naphthyridine-3-carboxylique,
le bromure de (±)-[3-éthoxycarbonyl-1,4-dihydro-5-isopropoxy-4-(2-trifluorométhylphényl)-1,6-naphthyridine-2-yl]-méthyl-1-pyridinium,
le chlorhydrate de l'éthylester d'acide (±)-2-(2-aminoéthoxyméthyl)-1,4-dihydro-5-isopropoxy-4-(2-trifluorométhylphényl)-1,6-naphthyridine-3-carboxylique,
l'éthylester de l'acide (±)-1,4-dihydro-5-isopropoxy-2-méthoxyméthyl-4-(2-trifluorométhylphényl)-1,6-naphthyridine-3-carboxylique,
l'éthylester de l'acide (±)-1,4-dihydro-2-hydroxyméthyl-5-isopropoxy-4-(2-trifluorométhylphényl)-1,6-naphthyridine-3-carboxylique,

l'éthylester de l'acide (±)-1,4-dihydro-5-isopropoxy-2-phénylthiométhyl-4-(2-trifluorométhylphényl)-1,6-naphthyridine-3-carboxylique,

l'éthylester de l'acide (±)-1,4-dihydro-5-isopropoxy-2-(4-pyridylthiométhyl)-4-(2-trifluorométhylphényl)-1,6-naphthyridine-3-carboxylique,

le 2-oxalate de l'éthylester de l'acide (±)-2-(2-N-benzyl-N-méthylaminoéthoxyméthyl)-1,4-dihydro-5-isopropoxy-4-(2-trifluorométhylphényl)-1,6-naphthyridine-3-carboxylique,

le dichlorhydrate du [2-(N-benzyl-N-méthyamino)éthyl]amide de l'acide (±)-1,4-dihydro-5-isopropoxy-2-méthyl-4-(2-trifluorométhylphényl)-1,6-naphthyridine-3-carboxylique,

le N-éthylamide de l'acide (±)-1,4-dihydro-5-isopropoxy-2-méthyl-4-(2-trifluorométhylphényl)-1,6-naphthyridine-3-carboxylique,

l'amide de l'acide (±)-1,4-dihydro-5-isopropoxy-2-méthyl-4-(2-trifluorométhylphényl)-1,6-naphthyridine-3-carboxylique,

l'éthylester de l'acide (±)-1,4-dihydro-5-isopropoxy-2-phénylsulfinylméthyl-4-(2-trifluorométhylphényl)-1,6-naphthyridine-3-carboxylique,

l'éthylester de l'acide (±)-1,4-dihydro-5-isopropoxy-2-phénylsulfonylméthyl-4-(2-trifluorométhylphényl)-1,6-naphthyridine-3-carboxylique,

le 2-aminoéthylester de l'acide (±)-1,4-dihydro-5-isopropoxy-2-méthyl-4-(2-trifluorométhylphényl)-1,6-naphthyridine-3-carboxylique,

l'éthylester de l'acide (±)-2-formyl-1,4-dihydro-5-isopropoxy-4-(2-trifluorométhylphényl)-1,6-naphthyridine-3-carboxylique,

l'éthylester de l'acide (±)-2-cyano-1,4-dihydro-5-isopropoxy-4-(2-trifluorométhylphényl)-1,6-naphthyridine-3-carboxylique,

le (±)-1,4-dihydro-5-isopropoxy-2-méthyl-4-(2-trifluorométhylphényl)-1,6-naphthyridine-3-carbonitrile,

le dichlorhydrate de l'éthylester de l'acide (±)-1,4-dihydro-5-(1-diméthylamino-2-propoxy)-2-méthyl-4-(2-trifluorométhylphényl)-1,6-naphthyridine-3-carboxylique,

l'éthylester de l'acide (±)-1,4-dihydro-2-(2-hydroxyéthoxyméthyl)-5-isopropoxy-4-(2-trifluorométhylphényl)-1,6-naphthyridine-3-carboxylique,

**2.** Dérivés de 5-oxysubstitués-1,6-naphtyridine, à savoir :

l'éthylester de l'acide (±)-2-2(2-diméthylaminoéthoxyméthyl)-1,4-dihydro-5-isopropoxy-4-(1-naphthyl)-1,6-naphthyridine-3-carboxylique,

l'éthylester de l'acide (±)-1,4-dihydro-5-isopropoxy-2-méthyl-4-(1-naphthyl)-1,6-naphthyridine-3-carboxylique,

le diméthylammonium bromure de (±)-[3-éthoxycarbonyl-1,4-dihydro-5-isopropoxy-4-(1-naphthyl)-1,6-naphthyridine-2-yl]méthyl-(2-hydroxyéthyl),

**3.** Dérivés de 5-oxysubstitués-1,6-naphtyridine, à savoir :

l'éthylester de l'acide (±)-2-dibromométhyl-1, 4-dihydro-5-isopropoxy-4-phényl-1,6-naphthyridine-3-carboxylique,

le méthylester de l'acide (±)-1,4-dihydro-5-isopropoxy-2-[(4-méthylpipérazine-1-yl)méthyl]-4-phényl-1,6-naphthyridine-3-carboxylique,

**4.** Dérivés de 5-oxysubstitués-1,6-naphtyridine, à savoir :

l'éthylester de l'acide (±)-4-(2-chlorophényl)-1, 4-dihydro-2-hydroxyméthyl-5-isopropoxy-1,6-naphthyridine-3-carboxylique,

le dichlorhydrate de l'éthylester de l'acide (±)-2-(2-aminoéthoxyméthyl)-4-(2-chlorophényl)-1,4-dihydro-5-isopropoxy-1,6-napthyridine-3-carboxylique, et

**5.** Ethylester d'acide (±)-4-(2-bromophényl) -2-dibromométhyl-1,4-dihydro-5-isopropoxy-1,6-naphthyridine-3-carboxylique.

**6.** Procédé de préparation de dérivés de 5-oxy-substitués 1,6-naphthyridine selon les revendications 1 à 5, caractérisé en ce que, soit

a) on procéde, d'une façon connue en soi, sur l'atome d'oxygène du lactame, à une alkylation dew dérivés de 1,6-naphthyridinène répondant à la formule générale V

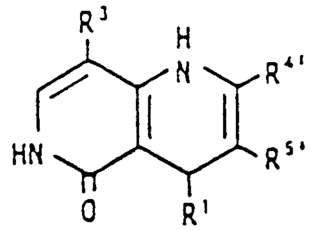

(V)

dans laquelle $R^1$ et $R^3$ qui, suivant les dérivés cités, présentent la signification correspondante, $R^{4'}$ représente un groupe méthyle ou un groupe amino et $R^{5'}$ signifie le reste méthoxycarbonyle, éthoxycarbonyle ou N-diphénylméthylpipérazinyléthoxycarbonyle ou un reste carboxamide représentant la formule générale IV

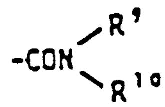

(IV)

dans laquelle $R^9$ et $R^{10}$ peuvent être identiques ou différents et représentent un atome d'hydrogène, un groupe éthyle ou un groupe N-benzyl-N-méthylaminoéthyle, et soit

b) on décompose des dérivés de 1,6-naphthyridine représentant la formule générale Ia

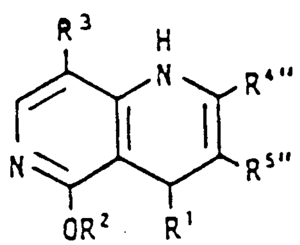

(Ia)

dans laquelle $R^1$ et $R^3$ présentent la signification indiquée ci-dessus, $R^2$ signifie un groupe alkyle à chaîne droite ou ramifiée renfermant jusqu'à 4 atomes de carbone, $R^{4''}$ représente un groupe méthyle et $R^{5''}$ représente un reste benzyloxycarbonyle d'une façon connue en soi, par hydrolyse, en acide carboxylique correspondant et on fait réagir ces dérivés amino d'une façon connue en soi, en passant par l'étape intermédiaire d'un halogénure d'acide, avec des alcools ou des amines substituées de façon correspondante en dérivés esters ou dérivés amides, des dérivés cités de 5-oxy-substitués-1,6-naphthyridine, soit

c) on procède à une monobromuration des dérivés de 1,6-naphthyridine de formule générale Ib

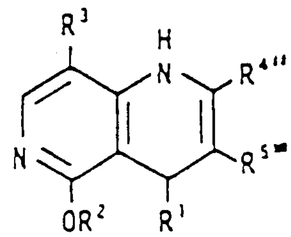

(Ib)

dans laquelle $R^1$, $R^2$, $R^3$ et $R^{4''}$ représentent la signification indiquée ci-dessus, et $R^{5'''}$ représente un reste méthoxycarbonyle, éthoxycarbonyle ou N-diphénylméthylpipérazinyléthoxycarbonyle ou un

reste carboxamide représentant de formule générarle IV

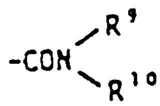

(IV)

dans laquelle $R^9$ et $R^{10}$ peuvent être identiques ou différents et signifient un atome d'hydrogène, un groupe éthyle ou un groupe N-benzyl-N-méthylaminoéthyl, on procède à une monobromuration sur le substituant $R^{4''}$ et, on les fait réagir avec des alcools des thiols ou des amines primaires, des amines secondaires et également des amines tertiaires substituées de façon correspondante et, le cas échéant, on fait passer ces produits par une oxydation appropriée en dérivés sulfinyles ou en dérivés sulfonyles correspondants, ou

d) on procède à une dibromuration des dérivés de la 1,6-naphthyridine de formule générale 1b sur le substituant $R^{4''}$ et l'on fait passer les dérivés dibromométhyl ainsi formés dans d'autres groupes fonctionnels tel qu'un groupe formyle ou groupe cyano, et soit

e) on procède, d'une façon connue en soi, à une alkylation en dérivés cités 5-oxy-substitués de 1,6-naphthyridine qui sont substitués en 2-méthyl-3-cyano des dérivés de 1,6-naphthyridinone de formule générale V que l'on obtient et dans lesquels $R^{5'}$ représente un groupe cyano et $R^{4'}$ représente un groupe méthyle par analogie avec les synthèses connues de dihydropyridine, on fait réagir avec la s-triazine les dérivés préparés répondant à la formule générale Va

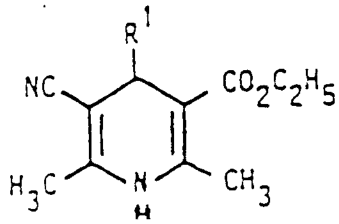

(Va)

dans laquelle $R^1$ présente la signification indiquée ci-dessus et en dérivés de formule générale I dans lesquels $R^5$ signifie un groupe cyano et $R^4$ représente un groupe méthyle.

f) on procède à une décomposition par hydrogénolyse d'une façon connue en soi des dérivés de 1,6-naphthyridine de formule générale I dans lesquels $R^5$ représente un reste N,N-dibenzylaminoalcoxycarbonyle pour obtenir des dérivés de formule générale I dans lesquels $R^5$ représente un groupe aminoalcoxycarbonyl non substitué ou un groupe aminoalkylcarbamoyl non substitué, ou

g) on procède à une décomposition par hydrogénolyse d'une façon connue en soi des dérivés de 1,6-naphthyridine de formule générale I dans lesquels $R^4$ représente un reste N-benzylaminoalcoxyméthyle, un reste N,N-dibenzylaminoalcoxyméthyle ou un reste azidoalcoxyméthyle pour obtenir des dérivés de formule générale I dans lesquels $R^4$ représente un reste aminoalcoxyméthyl non substitué.

**7.** Médicaments caractérisés en ce que, outre des dérivés auxiliaires et additifs usuels, ils contiennent des dérivés selon les revendications 1 à 5 pour le traitement des maladies vasculaires.

**8.** Procédé d'utilisation des dérivés selon les revendications 1 à 5 pour la préparation de médicaments destinés au traitement de maladies vasculaires.